# EUROPÄISCHE PATENTSCHRIFT

(11) **EP 0 335 231 B1**
(45) Veröffentlichungstag und Bekanntmachung des Hinweises auf die Patenterteilung: **14.07.1993**
(21) Anmeldenummer: 89105079.1
(22) Anmeldetag: 21.03.1989
(51) Int. Cl.: A61F 13/02

(54) **Pflaster, insbesondere Epikutanpflaster und Verfahren zur Herstellung desselben**
Plaster, in particular for epicutaneous test, and its method of fabrication
Tampon adhésif, en particulier pour tests épicutanés et son procédé de préparation

(30) Priorität: 29.03.1988 DE 3810658
(43) Veröffentlichungstag der Anmeldung: 04.10.1989
(73) Patentinhaber: Lohmann GmbH & Co. KG, 56567 Neuwied (DE)
(72) Erfinder: Anhäuser, Dieter, D-5451 Melsbach (DE); Seeger, Kurt, Dr., D-5450 Neuwied 12 (DE)
(74) Vertreter: Neidl-Stippler, Cornelia, Dr.

(56) Entgegenhaltungen:
- EP-A- 0 017 006
- EP-A- 0 117 632
- EP-A- 0 144 891
- DE-A- 3 827 431

## Beschreibung

Die Erfindung betrifft ein Epikutantestpflaster mit mindestens einer auf einer Trägerfolie angeordneten Wirkstoffaufnahmeeinrichtung sowie ein Verfahren zu seiner Herstellung.

Epikutanpflaster werden in Epikutantests eingesetzt, die insbesondere zur Aufklärung der Ursachen allergischer Kontaktekzeme dienen. Zu diesem Zweck wird die zu prüfende Testsubstanz auf ein Testpflaster aufgetragen, das dann auf geeignete Hautstellen des Patienten appliziert wird. Nach einem vorherbestimmten Zeitraum wird das Testpflaster abgezogen.

Die erste Ablesung der Reaktion des Patienten auf die Wirkstoffexposition erfolgt unmittelbar nach Abnahme der Testpflaster; weitere Ablesungen erfolgen später in bestimmten Zeitabständen. Dabei ist eine Markierungsmöglichkeit der Teststellen auf der Haut hilfreich, um die mit der Testsubstanz kontaktierte Hautstelle zuverlässig wiederfinden zu können.

Prinzipiell bauen sich Epikutantestpflaster wie folgt auf: Auf einer haftklebenden Trägerschicht sind als Wirkstoffaufnahmeeinrichtungen absorptionsfähige Flächengebilde, bspw. aus Stoff oder Vliesstoff, oder zur Haut hin offene Behältnisse angeordnet oder in dieser Trägerschicht ausgebildet, die die Testsubstanzen aufzunehmen vermögen. Die mit der Haut in Kontakt zu bringende Fläche des Testpflasters ist vor der Applikation durch eine ablösbare Schutzschicht abgedeckt. Als Trägerschicht wurden bisher textile Flächenmaterialien, wie beispielsweise Gewebe oder Vliesstoffe, Polymer- oder Metallfolien vorgeschlagen. Gattungsgemäße Pflaster sind aus der EP-A-0 017 006 bekannt. Der Nachteil dieser Materialien liegt darin, daß die Trägermaterialien eine zu geringe Elastizität besitzen, um das vorzeitige Ablösen des Testpflasters von der Haut durch unvermeidbare Körperbewegungen völlig zu unterbinden und durch Reibung an den Wirkstoffaufnahmeeinrichtungen hervorgerufene Reizungen, die auch eine allergische Reaktion vortäuschen können, zu vermeiden.

Weiterhin ist es insbesondere bei Pflastern, die einen längeren Zeitraum auf der Haut verbleiben, erwünscht, daß sie einerseits wasserdicht sind, um beim Waschen oder auch Duschen des Patienten nicht beeinträchtigt zu werden, andererseits wird Durchlässigkeit für Wasserdampf, um Mazeration der darunterliegenden Hautschicht zu vermeiden, gefordert. Dies konnte bislang noch nicht realisiert werden.

Aus der EP-A-0 144 891 ist ein Folienverband mit dünnen, Wasserdampf durchlässigen aber für flüssiges wasser dichten Folien bekannt.

Es ist daher Aufgabe der Erfindung, ein Pflaster, insbesondere Epikutanpflaster zu schaffen, das die geschilderten Mängel der bisherigen Lösungvorschläge vermeidet.

Die Lösung der Aufgabe ist in dem kennzeichnenden Teil des Anspruchs 1 angegeben.

Zur Erfüllung der Anforderungen an das Trägermaterial eines Epikutantestpflasters bezüglich Elastizität, Wasserdichtigkeit und Wasserdampfdurchlässigkeit bietet sich eine entsprechende Verminderung der Dicke der Polymerfolie an, doch scheiterte bisher die Verwendung extrem dünner Folien mit diesen erwünschten Eigenschaften bei dieser Art von Pflastern an der schlechten Handhabbarkeit dieser Folien. Sie sind außerordentlich wenig biegesteif, sozusagen lappig, knittern sehr leicht und neigen deshalb dazu, bei der Applikation auf sich selbst zu haften.

Um die Anwendbarkeit solcher Folien für Pflaster, insbesondere Epikutantestpflaster zu ermöglichen, wird erfindungsgemäß eine Flächenstabilisierung angewandt, bei der die extrem dünne Folie so lange durch Verbindung mit einer dickeren und steiferen zweiten Folie versteift wird, bis die Applikation erfolgt ist. Diese flächenstabilisierende Folie wird als Stützfolie bezeichnet. Sie hat mindestens die Größe der zu stabilisierenden Folie. Vorteilhaft ist es, sie an mindestens einer Seite über die zu stabilisierende Folie hinaus ragen zu lassen, um damit einen Anfaßrand für das spätere Abziehen der Stützfolie zu schaffen.

Bei einer weiteren bevorzugten Ausführung der Erfindung ragt die Stützfolie so weit über den Rand der Trägerfolie, daß parallel zu diesem Rand ein von der Trägerfolie getrennter haftklebender Markierstreifen Platz hat, der bei Applikation der Wirkstoffaufnahmeeinrichtung neben dieser auf der Haut klebt und nach Ablösen des Pflasters bevorzugt noch auf der Haut verbleibt und zur Markierung der Teststelle zur Verfügung steht.

Dieser Markierstreifen kann bspw. farbig ausgebildet sein Die Epikutantestpflaster nach der Erfindung können als Einzelpflaster, also nur miteiner Wirkstoffaufnahmeeinrichtung ausgebildet sein, weisen aber vorzugsweise mehrere nach einem vorherbestimmten geometrischen Muster, bevorzugt reihenförmig angeordnete Wirkstoffaufnahmeeinrichtungen auf. Zwischen den einzelnen Wirkstoffaufnahmeeinrichtungen können Sollbruchlinien vorgesehen werden, die eine leichte Abtrennung eines Pflasters mit der gewünschten Anzahl von Wirkstoffaufnahmeeinrichtungen von einer längeren Pflasterbahn ermöglichen.

Die gemäß der Erfindung einzusetzende wasserdampfdurchlässige und für flüssiges Wasser undurchlässige Trägerfolie weist ein geeignetes Polymer auf, wofür als Beispiel Polyurethane, Polyvinylchloride, Polyvinylidenchloride, Polyvinylalkohole, Polypropylen, Polyamide, Ethylen-Vinylacetat-Copolymere, Polyester, Polycarbonate, Polvinylfluorid und andere fluorhaltige Polymere genannt seien. Besonders bevorzugt sind Trägerfolien auf Polyurethanbasis. Die Schichtdicken geeigneter Trägerfolien bewegen sich im Bereich von 5 bis 120 µm, bevorzugt von 10 - 50 µm. Ihre Wasserdampfdurchlässigkeit soll mindestens 300 g/m² pro 24 Stunden betragen.

Die ein- oder mehrschichtige Stützfolie enthält vorzugsweise geeignete Polymere oder auch Metalle. Als geeignete Polymere seien beispielhaft Polyethylen, Polypropylen, Polyamide und Polyester angeführt. Gegebenenfalls sind diese Folien auf der Hautseite silikonisiert, um die erforderliche Trennkraft zwischen Trägerfolie und Stützfolie einzustellen. Die Folienstärke kann 20 - 200 µm, vorzugsweise 30 - 80 µm betragen. Der gegebenenfalls an der Stützfolie ausgebildete Anfaßrand sollte so breit sein, daß eine bequeme Handhabung möglich ist. Bevorzugt ist die Stützfolie mit der Trägerfolie nicht durch Klebung lösbar verbunden, sondern die Verbindung wird durch mechanischen Zusammenhalt bewerkstelligt, der bspw.dann entsteht, wenn die Trägerfolie durch Extrusion, Gießen oder eine andere bekannte Art der Folienherstellung unmittelbar auf der Stützfolie erzeugt wird. Bei Co-Extrusion beider Folien muß sogar umgekehrt darauf geachtet werden, daß die beiden Folien sich voneinander trennen lassen. Die Hautseite der Trägerfolie ist mit einer haftklebenden Schicht überzogen, wozu die bekannten physiologisch unbedenklichen Haftklebematerialien geeignet sind. Beispiele dafür sind Kautschuk, kautschukähnliche synthetische Homo-, Co- und Blockpolymere, Polyacrylate und deren Copolymerisate, Polyurethane und Silikone. Der Flächenauftrag des Haftklebers liegt zwischen 15 - 80 g/m², vorzugsweise zwischen 30 - 50 g/m².

Die Wirkstoffaufnahmeeinrichtungen sind vorzugsweise Abschnitte, wie kreisförmige Scheibchen oder andere Flächenformen aus absorptionsfähigem Material, wie beispielsweise Papier, Vliesstoff, Gewebe und gelartige Polymere, die den Wirkstoff an Haut abgeben können oder bestehen aus einem Schälchen zur Aufnahme des Wirkstoffes, wie einer Testsubstanz. Sie werden in an sich bekannter Weise auf der Haftklebeschicht befestigt. Um eine unbeabsichtigte Wanderung des flüssigen Wirkstoffes oder der Wirkstoffzubereitung aus den Wirkstoffaufnahmeeinrichtungen zu verhindern, können die Wirkstoffaufnahmeeinrichtungen mit einem derart dimensionierten Folienring abgedeckt werden, daß er mit etwa einer Hälfte seiner Breite die Wirkstoffaufnahmeeinrichtung abdeckt und mit der anderen Hälfte auf der Haftklebeschicht befestigt ist, ohne daß die vorgesehene Haut-Kontaktfläche der Wirkstoffaufnahmeeinrichtung beeinträchtigt wird.

Das Pflaster wird bevorzugt auf der haftklebenden Seite vor der Anwendung durch eine Schutzschicht abgedeckt. Diese Schutzschicht kann ein- oder mehrteilig sein, wobei aneinander grenzende Teile überlappen können, um eine Anfaßhilfe zur Entfernung der Schutzschicht zu bilden. Im Prinzip sind für die ggf. auch mehrschichtige Schutzschicht alle Materialien geeignet, die auch für die Stützfolie Anwendung finden. Darüberhinaus können aber auch beispielsweise Polytetrafluorethylen, Cellophan, Polyvinylchlorid, behandelte Papiere, Metallfolien und mit Polymeren überzogene Metallfolien eingesetzt werden. Die Flächengewichte liegen bei 50 - 200 g/m², vorzugsweise bei 80 bis 150 g/m². Die mit der Haftklebeschicht in Kontakt befindliche Seite der Schutzschicht muß eine Ablösung der Schutzschicht vom Rest des Pflasters mit einer Kraft erlauben, die kleiner ist als jene zur Ablösung der Stützfolie von der Trägerfolie benötigte.

Die Erfindung wird nachfolgend anhand der Figuren näher erläutert. Es zeigen in nicht maßstabsgetreuer Darstellung
- Fig.1:: schematisch einen Querschnitt durch eine bevorzugte Ausführungsform der Erfindung
- Fig.2:: schematisch eine Aufsicht von unten auf das Pflaster gemäß Fig. 1 ohne Schutzschicht,
- Fig.3:: schematisch einen Querschnitt durch eine weitere bevorzugte Ausführung eines Pflasters gemäß der Erfindung und
- Fig.4:: schematisch eine Aufsicht von unten auf ein Pflaster gemäß Fig. 3 ohne Schutzschicht.

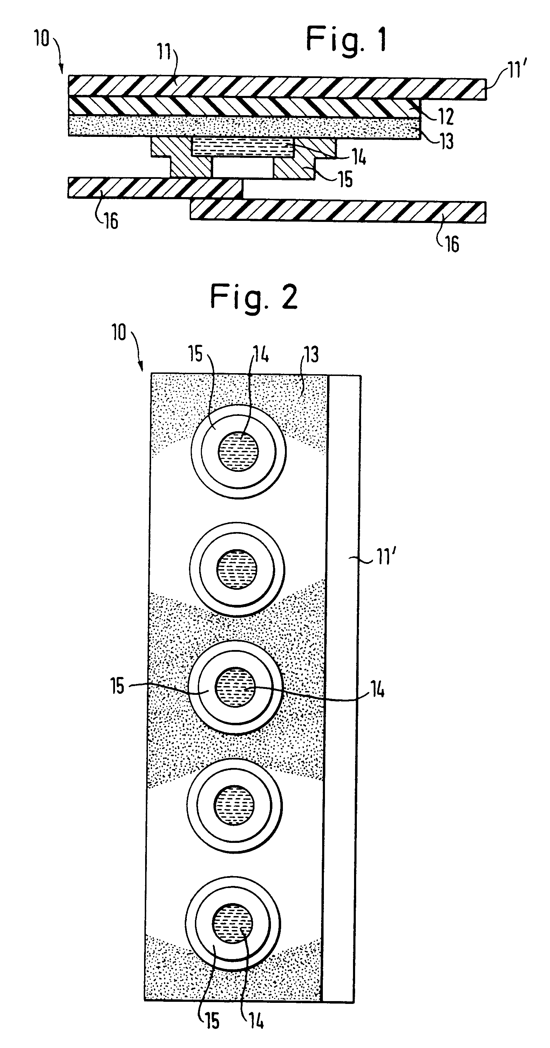

Der in Fig. 1 schematisch dargestellte Querschnitt einer bevorzugten Ausführungsform eines erfindungsgemäßen Epikutantestpflasters 10 zeigt eine Stützfolie 11, die die Trägerfolie 12 an einem Rand überragt und damit bei der Applikation einen Anfaßabschnitt bildet. Die Haftklebeschicht 13 besitzt dieselbe Dimensionierung wie die Trägerfolie 12. Die Wirkstoffaufnahmeeinrichtung 14 und der Sperring 15 sind auf der Haftklebeschicht befestigt. Eine zweiteilige Schutzschicht 16 deckt das gesamte Pflaster bis zur Applikation ab. Durch die Überlappung der beiden Teile der Schutzschicht wird eine Abziehhilfe gebildet.

Die Aufsicht auf die Hautseite eines erfindungsgemäßen Pflasters 10 bei abgezogener Schutzschicht läßt den Anfaßabschnitt 11' der Stützfolie 11 erkennen. Das Pflaster 10 umfaßt hier fünf reihenförmig angeordnete Wirkstoffaufnahmeeinrichtungen 14, die zusammen mit dem Sperring 15 auf der Haftklebeschicht 13 fixiert sind.

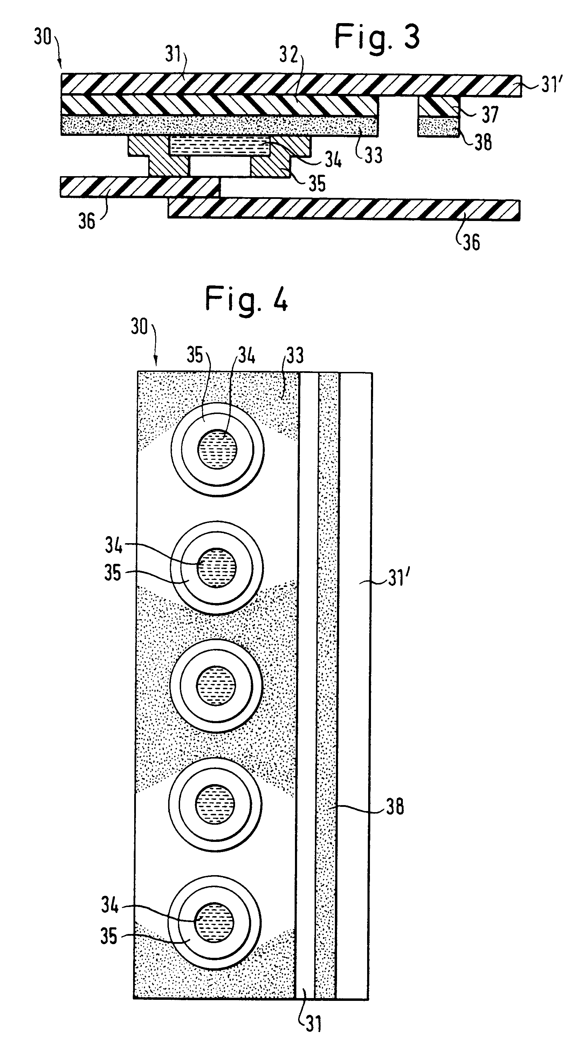

In Fig. 3 ist schematisch der Querschnitt durch eine weitere bevorzugte Ausführungsform der Erfindung wiedergegeben. Hier ist im überstehenden Rand 31'der Stützfolie 31 ein Markierungsstreifen 37 vorgesehen, der wie die Stützfolie 31 mit einer Klebeschicht 33 bedeckt ist. Der weitere Aufbau mit Wirkstoffaufnahmeeinrichtung 34, Sperrring 35 und Schutzschicht 36 entspricht der Fig. 1.

Die Fig. 4 zeigt eine Aufsicht auf die Hautseite des Epikutantestpflasters der Fig. 3 ohne Schutzschicht. Mit 31' ist der Anfaßrand der Stützfolie 31, mit 38 die durch den Zwischenraum zwischen haftklebender Trägerfolie 32 und Markierungsstreifen 37 sichtbare Hautseite der Kleberschicht, mit 34 die Wirkstoffaufnahmeeinrichtung und mit 35 der Sperring bezeichnet.

Die Herstellung der neuen, insbesondere als Epikutantestpflaster geeigneten Vorrichtungen kann erfindungsgemäß durch Erzeugen einer bahnförmigen Trägerfolie durch Aufbringen einer Polymerlösung unmittelbar auf einer bahnförmigen Stützfolie und Abdampfen des oder der Lösemittel erfolgen.. Eine andere bevorzugte Methode ist die Bildung eines Laminats aus Stützfolie und Trägerfolie durch Coextrusion. Hierbei müssen die Verfahrensparameter so eingestellt werden, daß die später erforderliche Trennbarkeit beider Folien gewährleistet ist. Auf die Hautseite, die Trägerfolie des derart erhaltenen Laminates wird nach bekannte Verfahren eine Haftklebeschicht bspw. im Transferverfahren aufgebracht, auf die in zwei zum Rand parallelen Reihen im gewünschtem Abstand scheibchenförmige Wirkstoffaufnahmeeinrichtungen befestigt werden. Diese werden sodann zentrisch mit Sperringen aus Folienmaterial versehen, wobei die die Wirkstoffaufnahmeeinrichtungen überragenden Ränder der Ringe auf der Haftklebeschicht fixiert werden. Parallel zu den Außenrändern der Bahn werden sodann durch eine tiefenkontrollierte Stanzung oder Schnit je ein Streifen aus Trägerfolie mit Haftkleber entfernt, so daß ein klebfreier Anfaßrand der Stützfolie entsteht. Zum Schutz des Pflasters läuft dann eine dreiteilige Schutzschicht über die Breite der Bahn zu, wobei entweder der mittlere Teil die beiden äußeren Teile überlappt oder die äußeren Teile den mittleren Teil überlappen, um die Wiederablösung vor Gebrauch zu erleichtern. Zur Konfektionierung wird die so erhaltene Laminatbahn mittig unter Bildung von zwei Epikutantestpflasterstreifen geteilt, die entsprechend der gewünschten Zahl von Wirkstoffaufnahmeeinrichtungren geteilt, die entsprechend der gewünschten Zahl der Wirkstoffaufnahmeeinrichtungen abgelängt werden. Sie können aber auch auf Rolle gewickelt oder in Leporello-Faltung abgelegt werden.

Vor dem Auftrennen der Breitware kann gewünschtenfalls quer zur Bahnrichtung in bestimmten Abständen eine Sollbruchlinie angebracht werden, die das Ablängen von Hand ohne zusätzliche Werkzeuge ermöglicht.

Zur Herstellung einer anderen bevorzugten Ausführungsform des Epikutantestpflasters wird bei tiefenkontrolliertem Stanzen oder Schneiden in dem oben geschilderten Verfahren ein von der Trägerfolie separierter noch auf der Stützfolie haftender Markierungsstreifen aus Trägerfolienmaterial und Haftkleber gebildet.

Das nachfolgende Beispiel erläutert das Herstellungsverfahren, die Erfindung ist jedoch keineswegs auf dieses begrenzt.

### Beispiel

Eine einseitig silikonisierte, 70 µm dicke, bahnförmige Polypropylen-Folie (1050 mm breit) wird mittels eines Walzenauftragwerkes mit einer Mischung aus 100 Gew.-Teilen einer 30 Gew.-%igen Lösung eines Polyesterurethanadduktes auf Basis Toluylendiisocyanat in Ethylacetat (Impranil C der Fa. Bayer), 7,5 Gew.-Teile einer 75 Gew.-%igen Läsung eines Vernetzers auf der Basis von Polyolen und aromatischem Diisocyanat in Ethylacetat (Imprafix TH der Fa. Bayer), 5 Gew.-Teile einer 10 Gew.-%igen Lösung eines Beschleunigers aus einem organischen Stickstoffderivat und einer Organometallverbindung in einem 1 : 4 : 4 -Gemisch aus Ethylacetat/Methylethylketon/Toluol (Imprafix BE der Fa. Bayer) und 75,7 Gew.-Teile Ethylacetat so beschichtet, daß nach dem Abdampfen der Lösemittel bei 30 - 70 °C eine 42 µm dicke Polyurethan-Folie ensteht.

Auf einem Silikonpapier wird dann mittels Walzenauftrag aus einer Mischung aus 100 Gew.-Teilen einer Acrylatlösung (Durotak 280 - 2516 der Fa. National Starch & Chemical BV) und 3 Gew.-Teilen Hercolyn der Fa. Hercules und anschließendes Abdampfen der Lösemittel bei 50 - 90 °C eine 40 µm dicke Haftklebeschicht erzeugt und auf das Laminat Stützfolie/Trägerfolie umkaschiert. Die soerhaltene Breitware wird auf 92 mm Breite aufgeschnitten.

Danach werden scheibchenförmige Wirkstoffaufnahmeeinrichtungen mit einem Durchmesser von 15 mm, die aus binderfreiem Vliesstoff (40% Zellwolle/60% Baumwolle, 80 g/m²) mit einseitiger Polyethylenbeschichtung ausgestanzt worden sind, in parallelen Reihen (Abstand 40 mm) im Abstand von 27,5 mm mit der Polyethylenseite auf die Haftklebeschicht aufgedrückt. Im nächsten Schritt werden die Wirkstoffaufnahmeeinrichtungen mit Sperringen aus Polyesterfolie versehen (Innendurchmesser 10 mm, Außendurchmesser 21 mm), wobei ein 6 mm breiter Rand auf der Klebeschicht befestigt ist.

Anschließend wird der Anfaßrand an der Stützfolie dadurch erzeugt, daß im Abstand von 10 mm parallel zu den Rändern der Bahn die Trägerfolie mit Haftklebeschicht durch einen tiefenkontrollierten Schneidevorgang durchtrennt wird und die entstehenden Streifen abgezogen werden.

Anschließend wird die Bahn mittig aufgetrennt, so daß zwei Epikutantestpflasterstreifen entstehen, die auf Rolle gewickelt werden.

## Patentansprüche

1. Epikutantestpflaster mit mindestens einer auf einer Trägerfolie (12,32) mit Haftklebeschicht (13) angeordneten Wirkstoffaufnahmeeinrichtung (14), dadurch gekennzeichnet, daß die Trägerfolie (12, 32) aus einem für flüssiges Wasser dichten, aber für Wasserdampf durchlässigen, hochelastischen Polymermaterial mit einer Schichtdicke von 5 - 120 µm besteht und an der der Haut abgewandten Fläche mit einer mindestens die Fläche der Trägerfolie abdeckenden Stützfolie (11, 31) ganzflächig oder an Teilflächenabschnitten lösbar verbunden ist, daß die Peripherie jeder Wirkstoffaufnahmeeinrichtung (14) durch einnen Sperring (15), der die Wirkstoffaufnahmeeinrichtung (14) allseitig überragt und auch auf der Haftklebeschicht fixiert ist, abgedeckt ist.

2. Pflaster nach Anspruch 1, dadurch gekennzeichent, daß die Stützfolie (11, 31) die Trägerfolie (12,32) an mindestens einem Rand überragt.

3. Pflaster nach einem der Ansprüche 1 oder 2, dadurch gekennzeichent, daß parallel zu mindestens einem Rand des Pflasters (10, 30) ein hautseitig haftklebender, mit hautabgewandter Fläche an der über die Trägerfolie hinausreichenden Stützfolie (11', 31') lösbar befestigter Markierungsstreifen (38) angeordnet ist.

4. Pflaster nach einem der Ansprüche 1 bis 3, dadurch gekennzeichnet, daß die Wirkstoffaufnahmeeinrichtungen (14, 34) reihenförmig angeordnet sind.

5. Pflaster nach einem oder mehreren der Ansprüche 1 bis 4, dadurch gekennzeichnet, daß zwischen den aneinandergereihten Wirkstoffaufnahmeeinrichtungen (14, 34) Sollbruchlinien vorgesehen sind.

6. Pflaster nach einem oder mehreren der Ansprüche 1 bis 5, dadurch gekennzeichnet, daß das die Trägerfolie (12,13) bildende Material ein Polymer auf Polyurethanbasis ist.

7. Verfahren zur Herstellung eines Pflasters nach einem oder mehreren der Ansprüche 1,2,4 und 6, gekennzeichnet durch folgende Schritte:
a) Erzeugen einer Trägerfolie auf einer Stützfolie durch: Aufbringen einer Trägerfolien-Polymerlösung auf eine bahnförmige Stützfolie und Abdampfen von Lösemittel oder Bildung eines bahnförmigen Laminats aus Stützfolie und Trägerfolie durch Coextrusion,
b) Aufbringen einer Haftkleberschicht auf die Hautseite der Trägerfolie des Laminates aus Stützfolie und Trägerfolie;
c) Aufbringen von Wirkstoffaufnahmeeinrichtungen auf die Haftklebeschicht nach einem vorherbestimmten Muster, bevorzugt in parallelen Reihen; wobei nach dem Aufbringen von Wirkstoffaufnahmeeinrichtungen auf die Haftklebeschicht zentrisches Abdecken der Wirkstoffaufnahmeeinrichtung mit Folienringen aus Polymermaterial erfolgt, und die die Wirkstoffaufnahmeeinrichtungen überragenden Ränder der Ringe auf der Haftklebeschicht fixiert werden.
d) Aufbringen einer mindestens einteiligen Schutzschicht auf das Laminat sowie
e) Abtrennen von Abschnitten, bevorzugt Streifen, entsprechend der gewünschten Anzahl von Wirkstoffaufnahmeeinrichtungen pro Pflaster oder Wickeln des Streifens auf Rolle oder Ablegen des Streifens in Leporellofaltung.

8. Verfahren zur Herstellung eines Pflaster nach Anspruch 7 dadurch gekennzeichnet, daß vor dem Aufbringen der Schutzschichten durch tiefenkontrolliertes Stanzen oder Schneiden des bahnförmigen laminatartigen Zwischenproduktes parallel zu den Außenrändern ein von der Trägerfolie abgetrennter, auf der Stützfolie haftender Streifen aus Trägerfolienmaterial mit Haftkleber gebildet wird.

9. Verfahren nach Ansprüche 7 oder 8, dadurch gekennzeichnet, daß das Aufbringen einer dreiteiligen Schutzschicht derart erfolgt, daß entweder der mittlere Teil die beiden äußeren Teile überlappt oder die äußeren Teile den mittleren Teil überlappen.

## Claims

1. Epicutaneous test plaster, having at least one active substance absorbing means (14), which is disposed on a carrier film (12, 32) having a layer of pressure sensitive adhesive (13), characterised in that the carrier film (12, 32) comprises a highly elastic polymer material, which is impermeable for liquid water but is permeable to water vapour, said material having a layer thickness of between 5 and 120 µm, and the face of the carrier film, remote from the skin, is detachably connected to a protective film (11, 31), which covers at least the area of the carrier film either over the entire area or at portions of the area, in that the periphery of each active substance absorbing means (14) is covered by a ring (15), which protrudes on all sides beyond the active substance absorbing means (14) and is also secured on the layer of pressure sensitive adhesive.

2. Plaster according to claim 1, characterised in that the protective film (11, 31) protrudes beyond the carrier film (12, 32) at at least one edge.

3. Plaster according to one of claims 1 or 2, characterised in that a marker strip (38), which adheres to the skin and is detachably fixed, by the surface remote from the skin, on the protective film (11', 31') extending beyond the carrier film, is disposed parallel to at least one edge of the plaster (10, 30).

4. Plaster according to one of claims 1 to 3, characterised in that the active substance absorbing means (14, 34) are arranged in rows.

5. Plaster according to one or more of claims 1 to 4, characterised in that predetermined breaking lines are provided between the active substance absorbing means (14, 34), which are arranged in rows relative to one another.

6. Plaster according to one or more of claims 1 to 5, characterised in that the material forming the carrier film (12, 13) is a polyurethane-based polymer.

7. Method of producing a plaster according to one or more of claims 1, 2, 4 and 6, characterised by the following steps:
a) producing a carrier film on a support film by: applying a carrier film polymer solution to a wab-like support film and evaporating the solvent or forming a web-like laminate from support film and carrier film by coextrusion;
b) applying a layer of pressure sensitive adhesive to the skin side of the carrier film of the laminate formed from support film and carrier film;
c) applying active substance absorbing means to the layer of pressure sensitive adhesive according to a predetermined pattern, preferably in parallel rows, whereby, after applying active substance absorbing means to the layer of pressure sensitive adhesive, the active substance absorbing means is centrally covered with film rings formed from polymeric material, and the edges of the rings protruding beyond the active substance absorbing means are fixed on the layer of pressure sensitive adhesive;
d) applying a protective layer, at least a one-piece protective layer, to the laminate; and
e) separating portions, preferably strips, according to the desired number of active substance absorbing means per plaster, winding the strip into a roll or depositing the strip so that it is folded in Leporello manner.

8. Method of producing a plaster according to claim 7, characterised in that, before the protective layers are applied by depth-controlled punching or before the web-like, laminate-like intermediate product is cut parallel to the external edges, a strip of carrier film material, which has been fixed from the carrier film and adheres on the support film, is formed with pressure sensitive adhesive.

9. Method according to claims 7 or 8, characterised in that a three-part protective layer is applied in such a manner that either the central portion overlaps the two outer portions or the outer portions overlap the central portion.

## Revendications

1. Tampon pour tests épicutanés comportant au moins un dispositif récepteur de principe actif (14) agencé sur une feuille de support (12, 32) pourvue d'une couche adhésive (13), caractérisé en ce que la feuille de support (12, 32) est constituée d'une matière polymère très élastique imperméable à l'eau liquide, mais perméable à la vapeur d'eau, d'une épaisseur de 5 à 120 micromètres et est reliée de manière détachable sur la surface opposée à la peau, sur la totalité de la surface ou sur des sections partielles de surface, à une feuille de soutien (11, 31) recouvrant au moins la surface de la feuille de support, la périphérie de chaque dispositif récepteur de principe actif (14) est recouverte par un anneau de blocage (15), qui dépasse de tous côtés le dispositif récepteur de principe actif (14) et est fixé également sur la couche adhésive.

2. Tampon selon la revendication 1, caractérisé en ce que la feuille de soutien (11, 31) dépasse la feuille de support (12, 32) sur au moins un bord.

3. Tampon selon la revendication 1 ou 2, caractérisé en ce qu'une bande de marquage (38) adhésive côté peau et fixée de manière détachable à la surface opposée à la peau sur la feuille de soutien (11', 31') appliquée sur la feuille de support est agencée parallèlement à au moins un bord du tampon (10, 30).

4. Tampon selon l'une quelconque des revendications 1 à 3, caractérisé en ce que les dispositifs récepteurs de principe actif (14, 34) sont agencés en rangée(s).

5. Tampon selon une ou plusieurs des revendications 1 à 4, caractérisé en ce que des lignes de rupture sont prévues entre les dispositifs récepteurs de principe actif (14, 34) agencés en rangées.

6. Tampon selon une ou plusieurs des revendications 1 à 5, caractérisé en ce que le matériau constituant la feuille de support (12, 13) est un polymère à base de polyuréthane.

7. Procédé de fabrication d'un tampon selon une ou plusieurs des revendications 1, 2, 4 et 6, caractérisé par les étapes suivantes :
a) On produit une feuille de support sur une feuille de soutien par application d'une solution de polymère de la feuille de base sur une feuille de soutien en forme de ruban et on évapore le solvant ou on forme un stratifié en forme de ruban à partir d'une feuille de soutien et d'une feuille de support par coextrusion,
b) On applique une couche adhésive sur le coté peau de la feuille de support du stratifié constitué de la feuille de soutien et de la feuille de support,
c) On applique des dispositifs récepteurs de principe actif sur la couche adhésive selon un motif prédéterminé, de préférence en rangées parallèles, les dispositifs récepteurs de principe actif étant revêtus au centre par des anneaux en feuille constitués de matière polymère après l'application desdits dispositifs récepteurs de principe actif sur la couche adhésive et les bords des anneaux dépassant des dispositifs récepteurs de principe actif étant fixés sur la couche adhésive,
d) On applique une couche protectrice au moins d'un seul tenant sur le stratifié, et
e) On sépare des sections, de préférence des bandes, selon le nombre souhaité de dispositifs récepteurs de principe actif par tampon ou on enroule la bande en rouleau ou encore on dispose la bande en accordéon par pliage.

8. Procédé de préparation d'un tampon selon la revendication 7, caractérisé en ce que, avant d'appliquer les couches protectrices, on forme par échenillage, c'est-à-dire par découpe contrôlée en profondeur, ou par section du produit intermédiaire stratifié en forme de bande, parallèlement aux bords externes, une bande de matière de feuille de support pourvue d'un adhésif, séparée de la feuille de support et collée à la feuille de soutien.

9. Procédé selon la revendication 7 ou 8, caractérisé en ce que l'application d'une couche protectrice triple se fait en sorte que soit la partie centrale chevauche les deux parties externes, soit les parties externes chevauchent la partie centrale.
